# EUROPEAN PATENT APPLICATION

(11) **EP 4 495 599 A1**
(43) Date of publication of application: **22.01.2025**
(21) Application number: 23769971.5
(22) Date of filing: 16.03.2023
(51) Int. Cl.: G01N 33/68, C07K 14/47

(54) **METHOD AND KIT FOR DIAGNOSING ALZHEIMER'S DISEASE BASED ON THE DETECTION OF APOLIPOPROTEIN E**

(30) Priority: 17.03.2022 ES 202230224
(71) Applicant: Universidad Miguel Hernández de Elche, 03202 Elche, Alicante (ES); Consorcio Centro de Investigación Biomédica en Red, 28029 Madrid (ES)
(72) Inventor: SÁEZ VALERO, Javier, 03550 San Juan de Alicante Alicante (ES); LENNOL, Matthew P., 03550 San Juan de Alicante Alicante (ES); CUCHILLO IBÁÑEZ, Inmaculada, 03550 San Juan de Alicante Alicante (ES)
(74) Representative: Manuel Illescas y Asociados, S.L.
(86) International application number: PCT/ES2023/070162
(87) International publication number: WO 2023/175225

(57) **Abstract**

The present invention relates to an *in vitro* method for the diagnosis of Alzheimer's disease, comprising the determination of an apolipoprotein E (ApoE)-based biomarker selected from the group consisting of: presence, amount, or concentration ApoE having a size of 34 kDa in an ApoE aggregate having a size of 100 kDa; ratio of ApoE dimers/monomers detected in a native electrophoresis assay; and presence of ApoE dimers detected in a native electrophoresis assay. The present invention also relates to a kit for diagnosing Alzheimer's disease comprising reagents for carrying out the method of the invention.

## Description

### TECHNICAL FIELD OF THE INVENTION

The invention relates to *in vitro* diagnostic methods in humans in which a biomarker for Alzheimer's disease is determined, and to diagnostic kits comprising reagents for the determination of biomarkers of Alzheimer's disease.

### BACKGROUND OF THE INVENTION

Alzheimer's disease (AD) is a global health problem that has an increasingly important burden on society as life expectancy increases.

Apolipoprotein E (ApoE) is a component of lipoprotein particles in plasma, as well as in cerebrospinal fluid (CSF) and the interstitial fluid of the brain parenchyma in the central nervous system (CNS). It plays a role in the transport of lipids, including cholesterol, and also participates in various other functions beyond being a part of lipoproteins, in part by interacting with cell signal transduction receptors. In the CNS, ApoE is mostly secreted by astrocytes and its receptors are neuronal and microgial.

The ε4 allele of ApoE has been identified as a very important risk factor for AD. Humans are the only species with three versions or isoforms of the *APOE* gene, ε2 allele (ApoE2), ε3 allele (ApoE3) and ε4 allele (ApoE4); other mammals have only one version of the *APOE* gene.

Differences in the structure of ApoE isoforms influence their ability to bind to lipids, but also to receptors and to the triggering effector of AD, the β-amyloid peptide (Aβ). These differences in their interactions with various molecules may be due to the fact that while ApoE2 and ApoE3 have a Cys residue at position 112, allowing them to form disulphide-linked heterodimers and homodimers, the ApoE4 isoform has Arg at that position, and consequently cannot form disulphide-linked dimers.

Carrying one ε4 allele implies a 2-3-fold increase in the probability of developing AD and greater than 8-10 times if both ε4 alleles are present; while carrying the ε2 allele is protective against the development of AD. About 75% of the population are carriers of ApoE3, considered risk-neutral. Carriers of ApoE4 make up around 15% of the population, while the presence of ApoE2 is relatively rare, with an incidence of less than 5% in the population. Due to its high association with AD, the percentage of ApoE4 carriers is around 35-40% in patients with this disease.

In the state of the art, methods have been described for determining the type of ApoE variant, as well as methods for sub-grouping AD patients by *APOE* genotypes to estimate other biomarkers.

However, methods or kits for diagnosing AD based on alterations of the ApoE protein (e.g., aberrant or abnormal forms of ApoE) associated with the development of AD have not been described in the state of the art.

Despite advances in AD diagnosis, there is a need to develop AD diagnostic methods and kits based on new biomarkers.

### DESCRIPTION OF THE INVENTION

For the purposes of the present invention, "subject" refers to a human being. More preferably, said subject has Alzheimer's disease or is suspected of having, or is at risk of having, said disease. Subjects who are affected by said disease can be identified by the symptoms that accompany the disease, which are known in the state of the art. However, a subject suspected of being affected by the aforementioned disease may also be an apparently healthy subject, for example, one being investigated during a routine clinical examination, or may be a subject at risk of developing the aforementioned disease.

For the purposes of the present invention, the term "sample" refers to a sample of a bodily fluid, a sample of cells, a tissue sample, or a wash/rinse fluid sample obtained from an external or internal body surface. Preferably, the samples are samples of cerebrospinal fluid, urine, blood, whole blood, plasma, serum, tear, lymphatic fluid, saliva, cells and tissues.

For the purposes of the present invention, the term "comparing" refers to contrasting the determination of the ApoE-based biomarker in the sample to be analysed, against the determination of the biomarker, in samples from appropriate reference subjects, as specified hereinafter. The aforementioned comparison can be carried out manually or be computer-assisted. In the case of a computer-assisted comparison, the value of the determined quantity may be compared with values corresponding to suitable references that are stored in a database using a computer software. Accordingly, the result of the identification referred to herein may be automatically provided in a suitable output format.

For the purposes of the present invention, "reference subjects" are healthy subjects, for whom it is known that they do not suffer from Alzheimer's disease. The determination of the ApoE-based biomarker can be carried out, using the methods of the present invention, from a sample of reference subjects to be analysed either simultaneously or subsequently with the test sample. A cut-off value may be used as a reference value associated with the biomarker in reference subjects.

For the purposes of the present invention, the term "indicative" refers to:
(a) the presence, or an increased amount or concentration of ApoE with a size of 34 kDa in an ApoE aggregate with a size of 100 kDa, in the biological sample to be analysed, relative to the reference subjects; or
(b) a higher dimer/monomer ratio of ApoE detected by a native electrophoresis assay, in the biological sample to be analysed, relative to the reference subjects; or
(c) the presence of ApoE dimers detected by a native electrophoresis assay, in the biological sample to be analysed;
allows diagnosing whether a subject has Alzheimer's disease.

For the purposes of the present invention, "lectin" refers to proteins that specifically bind to sugars.

For the purposes of the present invention, "native electrophoresis" refers to an electrophoresis assay on a native gel, also referred to as a non-denaturing gel. In this technique, proteins are analysed in their folded state and, therefore, electrophoretic mobility depends not only on the charge-to-mass ratio, but also on the shape and size of the protein.

For the purposes of the present invention, "Western blot", also referred to as "immunoblot" or "electrotransfer", refers to an analytical technique used in cell and molecular biology to identify specific proteins in a complex mixture of proteins, such as that present in cell or tissue extracts. The technique uses the following three steps: separation by size, transfer to a solid support, and finally visualization by binding proteins to appropriate primary or secondary antibodies.

For the purposes of the present invention, the term "ELISA" refers to enzyme-linked immunoabsorbent assay, which is an immunoassay technique in which an immobilized antigen is detected by an antibody bound to an enzyme (peroxidase, alkaline phosphatase, etc.,) capable of generating a detectable product from a substrate by a colour change or some other type of change caused by the enzymatic action on said substrate. In said technique, there may be a primary antibody that recognizes the antigen and that in turn is recognized by a secondary antibody bound to said enzyme. The antigen can be detected indirectly in the sample by colour changes measured by spectrophotometry.

The technical problem to be solved consists of the development of an *in vitro* method and a kit for the diagnosis of AD based on new biomarkers.

This invention, defined by the object of the claims, provides a solution to said technical problem.

The present invention provides an *in vitro* method for diagnosing Alzheimer's disease in a subject, comprising:
(a) determining an apolipoprotein E (ApoE)-based biomarker selected from the group consisting of:
   (i) presence, amount or concentration of ApoE with a size of 34 kDa in an aggregate of ApoE with a size of 100 kDa;
   (ii) dimer/monomer ratio of ApoE detected in a native electrophoresis assay, in subjects with an ApoE3/4 genotype; and
   (iii) presence of ApoE dimers detected in a native electrophoresis assay, in subjects with an ApoE4/4 genotype; and
(b) comparing the result of the determination of step (a) with a result obtained in reference subjects, wherein any of the following conditions is indicative of a positive diagnosis of Alzheimer's disease in said subject:
   (i) the presence, or an increased amount or concentration of ApoE with a size of 34 kDa in an aggregate of ApoE with a size of 100 kDa, in said biological sample, relative to the reference subjects;
   (ii) a higher dimer/monomer ratio of ApoE detected by a native electrophoresis assay, in said biological sample, relative to the reference subjects;
   (iii) the presence of ApoE dimers detected by a native electrophoresis assay, in said biological sample.

The biomarker of step (a)(i) of the method of the invention is based on the fact that 34 kDa ApoE is an aberrant species of ApoE that is part of larger aggregates of 100 kDa and that said aberrant species is associated with the development of AD.

The biomarker of step (a)(ii) of the method of the invention is based on the fact that the ratio of ApoE dimers/monomers detected in a native electrophoresis assay is associated with the development of AD in subjects with an ApoE3/4 genotype.

The biomarker of step (a)(iii) of the method of the invention is based on the fact that the ApoE dimers detected in a native electrophoresis assay is an aberrant species of ApoE associated with the development of AD in subjects with an ApoE4/4 genotype.

In a preferred embodiment of the method of the invention, in step (a) ApoE with a size of 34 kDa is detected with a lectin specific for ApoE with a size of 34 kDa. ApoE is a glycosylated protein and can be detected with a specific lectin (the lectin recognizes the sugars present in ApoE).

Such specific lectin includes, but not limited to:
(a) sialic acid-specific lectin including, but not limited to: *Sambucus nigra* lectin (SNA), *Maackia amurensis* lectin (MAL), *Maackia amurensis* lectin (MAA), *Limax flavus* lectin, Allomyrin *dichotoma lectin* and sialic l1 lectin;
(b) sialic acid and N-acetyl-β-D-glucosamine specific lectin including, but not limited to, *Triticum vulgaris* wheat germ agglutinin (WGA);
(c) mannose-specific lectin including, but not limited to: *Lens culinaris* lectin (LCA) and concanavalin A (Con A);
(d) N-acetylgalactosamine-specific lectin including, but not limited to: *Griffonia simplicifolia* lectin (GSL); *Helix aspersa letin* (HAA), *Helix pomatia* lectin (HPA), *Vicia villosa* lectin (VV), soy agglutinin (SBA) and *Sophora japonica* lectin (SJL); and
(e) oligosaccharide-specific lectin including, but not limited to, *Phaseolus vulgaris* lectin (PHA).

In another preferred embodiment of the method of the invention, in step (a) the presence of ApoE4 is detected in the ApoE aggregate with a size of 100 kDa, in a biological sample from a subject with an APOE3/4 or APOE4/4 genotype, with the presence of ApoE4 in the ApoE aggregate with a size of 100 kDa being indicative of a positive diagnosis of Alzheimer's disease in said subject.

The vast majority of subjects with the APOE4/4 genotype suffer from AD. However, such subjects cannot be treated for AD prior to the onset of clinical symptoms. It is desirable to have an early diagnosis before the onset of clinical symptoms in order to begin treating the disease as soon as possible. The preferred embodiment of the preceding paragraph provides a diagnostic method for subjects with the ApoE4/4 genotype, enabling the early diagnosis of AD before the onset of clinical symptoms, thereby allowing the initiation of treatment for the disease before clinical symptoms appear.

In a more preferred embodiment of the method of the invention, ApoE4 is detected with an ApoE4-specific antibody. Several commercial ApoE4-specific antibodies exist, including, but not limited to: NBP1-49529 antibody (Novus Biologicals), EPR24181-64 antibody (Abcam), MABN43 antibody (Sigma-Aldrich), and others. In a further preferred embodiment of the method of the invention, said ApoE4-specific antibody is the NBP1-49529 antibody, the EPR24181-64 antibody, and the MABN43 antibody.

In another preferred embodiment of the method of the invention, in step (a) ApoE is detected with an antibody specific for all isoforms of ApoE. There are numerous commercially available antibodies specific for all isoforms of ApoE, including, but not limited to: the AB178479 antibody (Merck Millipore), the AB947 antibody (Merck Millipore), and others. Preferably, said antibody specific for all isoforms of ApoE is the AB178479 antibody or the AB947 antibody.

In another preferred embodiment of the method of the invention, said biological sample is selected from the group consisting of: cerebrospinal fluid, urine, blood, whole blood, plasma, serum, tear, lymphatic fluid, saliva, cells and tissues.

In another preferred embodiment of the method of the invention, in the determination of the biomarker in step (a)(i), a technique selected from the group consisting of: gel electrophoresis under reducing conditions, gel electrophoresis under non-reducing conditions, sodium dodecyl sulphate polyacrylamide gel electrophoresis (SDS-PAGE) under reducing conditions, SDS-PAGE under non-reducing conditions, native electrophoresis, native polyacrylamide gel electrophoresis (PAGE), Western blot, lectin binding assays and enzyme-linked immunoabsorption assay (ELISA) is used. Preferably, gel electrophoresis under reducing conditions comprises using the reducing agent β-mercaptoethanol or dithiothreitol.

In another preferred embodiment of the method of the invention, in the determination of the biomarker in step (a)(ii) or (a)(iii), the assay is a native polyacrylamide gel electrophoresis assay (PAGE).

The present invention also provides a kit for diagnosing Alzheimer's disease, comprising reagents for:
(a) detecting and/or quantifying the amount or concentration of ApoE with a size of 34 kDa in an aggregate of ApoE with a size of 100 kDa, in a biological sample obtained from a subject; or
(b) determining the ratio of ApoE dimers/monomers detected in a native electrophoresis assay, in a biological sample obtained from a subject; or
(c) detecting the presence of ApoE aggregates detected in a native electrophoresis assay, in a biological sample obtained from a subject, wherein the kit comprises at least the AB178479 antibody or the AB947 antibody, specific for all isoforms of ApoE.

There are numerous commercially available antibodies specific for all isoforms of ApoE, including, but not limited to: the AB178479 antibody (Merck Millipore), the AB947 antibody (Merck Millipore), and others.

In a preferred embodiment, the kit of the invention additionally comprises a lectin specific for ApoE with a size of 34 kDa.

In another preferred embodiment, the kit of the invention additionally comprises an ApoE4-specific antibody selected from the group consisting of: the NBP1-49529 antibody, the EPR24181-64 antibody, and the MABN43 antibody. Several commercial ApoE4-specific antibodies exist, including, but not limited to: NBP1-49529 antibody (Novus Biologicals), EPR24181-64 antibody (Abcam), MABN43 antibody (Sigma-Aldrich), and others.

In another preferred embodiment, the kit of the invention additionally comprises the reducing agent β-mercaptoethanol or dithiothreitol.

In another preferred embodiment, the kit of the invention additionally comprises at least one buffer solution.

Throughout the description and the claims, the term "comprising", "that comprises" and their variants are meant in a non-limiting sense and therefore should not exclude other technical features. The term "comprises", "comprising" and its variants, throughout the description and claims, specifically includes the term "consists of", "consisting of" and their variants.

As used herein and in the claims, the singular form "the" includes references to plural forms unless the content clearly indicates otherwise.

Unless defined otherwise, all the technical and scientific terms used throughout the description and claims have the same meaning as those customarily understood by a person skilled in the field of the invention.

### BRIEF DESCRIPTION OF THE DRAWINGS

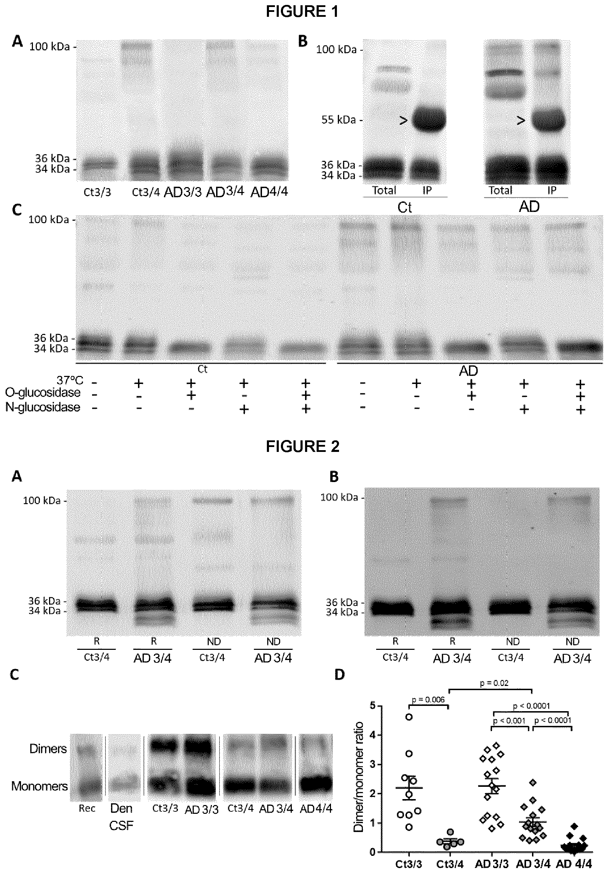

Figure 1. Characterization of the ApoE protein in cerebrospinal fluid (CSF) samples from controls or patients with AD. (A) CSF samples from controls (represented by the label "Ct") and AD cases with different APOE genotypes (ε3/ε3: 3/3; ε3/ε4: 3/4; ε4/ε4: 4/4) were immunoblotted with ApoE antibody AB178479. (B) The CSF samples were immunoprecipitated with the ApoE antibody AB178479 and the immunoprecipitated proteins were immunoblotted with the ApoE antibody AB947 (labelled "IP") >: indicates a non-specific band. (C): The CSF samples were immunoblotted with the ApoE antibody AB178479 after O-linked deglycosylation, N-linked deglycosylation or the combination of both. Samples heated to 37°C in the absence of deglycosylating enzymes were included as a control of the deglycosylation process.
Figure 2. Characterization of ApoE species in CSF by sodium dodecyl sulphate polyacrylamide gel electrophoresis (SDS-PAGE) and native polyacrylamide gel electrophoresis (PAGE). CSF samples from controls (labelled "Ct") and AD cases with different APOE ε3/ε4 genotypes (3/4) were immunoblotted after non-reducing (NR) or reducing (R) SDS-PAGE with (A) ApoE antibody AB178479 or (B) ApoE4-specific antibody NBP1-49529. (C) Representative transfer of CSF samples (ε3/ε3: 3/3; ε3/ε4: 3/4; ε4/ε4: 4/4) resolved by native PAGE with AB178479 antibody, revealing ApoE monomers (~35 kDa) and dimers (~70 kDa). Recombinant ApoE protein samples (tagged "Rec") and a control CSF sample denatured by boiling at 95°C for 5 min (tagged "den. LCR") were used to identify the positions of monomeric and dimeric ApoE bands. (D) Quantification of the dimer/monomer ratio of ApoE in the different APOE genotypes. The figure shows means ± standard error and p-values.

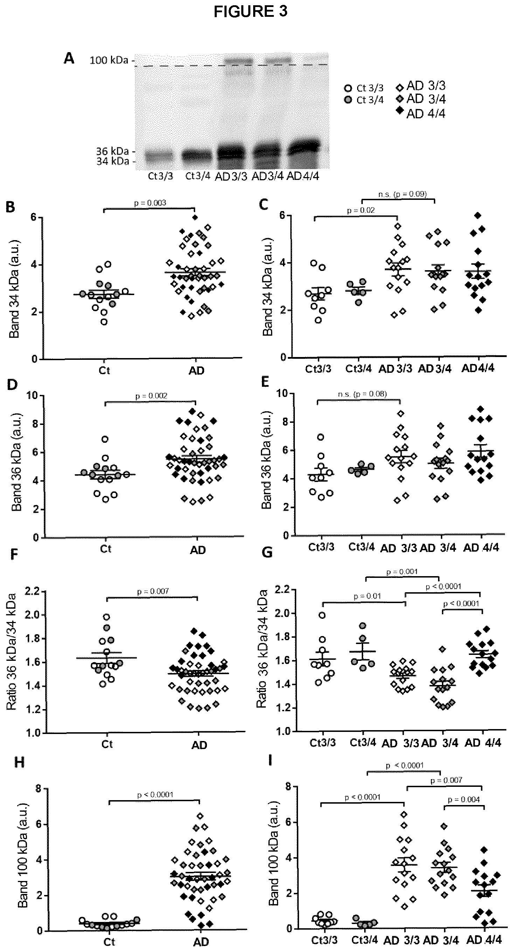

Figure 3. Analysis of ApoE species in CSF from the Gothenburg cohort. CSF samples from controls (represented by the tag "Ct") and AD cases with different APOE genotypes (ε3/ε3: 3/3; ε3/ε4: 3/4; ε4/ε4: 4/4) were analysed by Western blot with the AB178479 antibody after SDS-PAGE. (A) Representative immunoblots are shown. (B) Densitometric quantification of the 34 kDa ApoE immunoreactive band in control and AD cases. (C) Subgroup representation by APOE genotypes of the cases represented in (B). (D) Densitometric quantification of 36 kDa ApoE in control and AD cases. (E) Subgroup representation by APOE genotypes of the cases represented in (D). (F) ApoE 36kDa/34kDa ratio in each sample for control and AD cases. (G) Subgroup representation by APOE genotypes of cases represented in (F). (H) Densitometric quantification of 36 kDa ApoE in control and AD cases. (I) Representation of subgroups by APOE genotypes of the cases represented in (H). Each individual band was quantified and normalized to the reference value (recombinant ApoE). The figure shows means ± standard error and p-values.

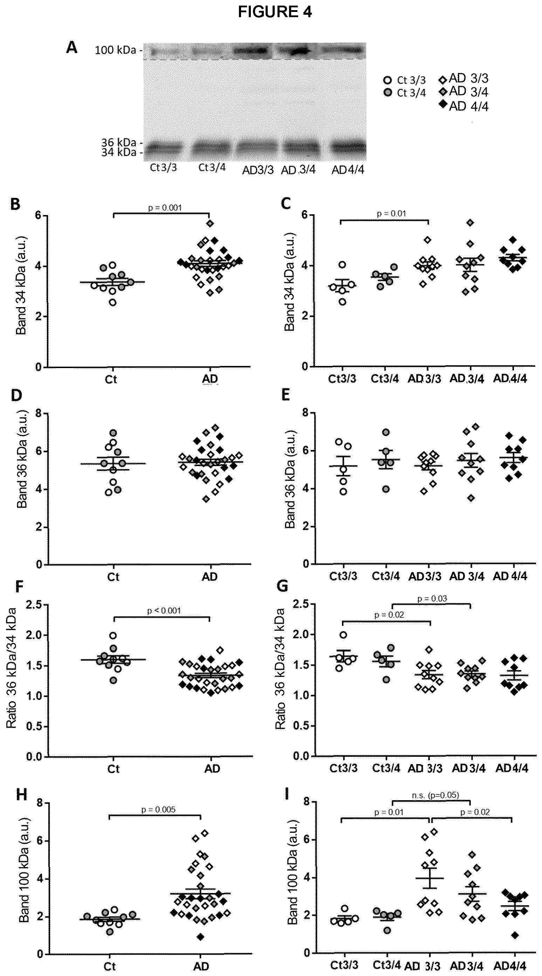

Figure 4. Analysis of ApoE species in CSF from the Barcelona cohort. CSF samples from controls (represented by the tag "Ct") and AD cases with different APOE genotypes (ε3/ε3: 3/3; ε3/ε4: 3/4; ε4/ε4: 4/4) were analysed by Western blot with the AB178479 antibody after SDS-PAGE. (A) Representative immunoblots are shown. (B) Densitometric quantification of the 34 kDa ApoE immunoreactive band in control and AD cases. (C) Subgroup representation by APOE genotypes of the cases represented in (B). (D) Densitometric quantification of 36 kDa ApoE in control and AD cases. (E) Subgroup representation by APOE genotypes of the cases represented in (D). (F) ApoE 36kDa/34kDa ratio in each sample for control and AD cases. (G) Subgroup representation by APOE genotypes of cases represented in (F). (H) Densitometric quantification of 36 kDa ApoE in control and AD cases. (I) Representation of subgroups by APOE genotypes of the cases represented in (H). Each individual band was quantified and normalized to the reference value (recombinant ApoE). The figure shows means ± standard error and p-values.

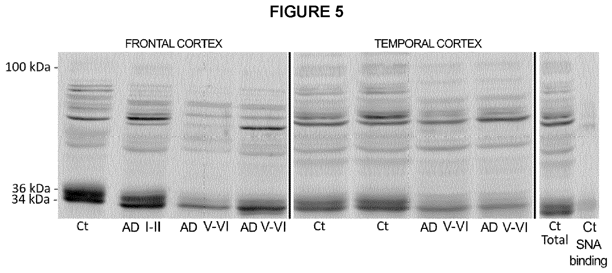

Figure 5. Analysis of two different collections of extracts of frontal cortex and temporal cortex from brains of controls and patients with AD by Western blot. On the right, the results of a binding assay to SNA lectin (*Sambucus nigra* lectin specific for sialic acid) bound to agarose in brain samples from control subjects are shown. The total (tagged "Ct Total") and the lectin-bound fraction (tagged "Ct SNA Binding") are shown.

### DESCRIPTION OF EMBODIMENTS

### Materials and methods

### Patients

Lumbar cerebrospinal fluid (CSF) samples with known APOE genotypes were obtained from two independent cohorts. The CSF samples from both cohorts used in this study were anonymised aliquots from routine clinical analyses, following a procedure approved by the Ethics Committees of the University of Gothenburg and Sant Pau Hospital, respectively. This study was approved by the Miguel Hernández University Ethics Committee and was conducted in accordance with The Declaration of Helsinki on Human Research. The first cohort was from the Clinical Neurochemistry Laboratory (Gothenburg, Sweden) and included patients who sought clinical advice regarding minor clinical symptoms. This first cohort consisted of 45 patients with AD (14 men and 31 women, mean age 77 ± 1 years; APOE: 15 ε3/ε3, 15 ε3/ε4, 15 ε4/ε4) and 14 controls without AD (7 men and 7 women, mean age (67± 3 years); APOE: 9 ε3/ε3, 5 ε3/ε4). The second cohort was obtained from Hospital Sant Pau (Barcelona, Spain). This second cohort consisted of samples from 29 patients with AD (13 men and 16 women, mean age 73 ± 1 years; APOE: 10 ε3/ε3, 10 ε3/ε4, 9 ε4/ε4) and 10 controls (7 men and 3 women, mean age 69 ± 2 years; APOE: 5 ε3/3, 5 ε3/4).

Patients were designated as AD or controls according to CSF biomarker levels using cut-offs that are >90% specific for AD: Aβ42 < 550 ng/L and total tau (T-tau) > 400 ng/L.). All patients with AD met the NIA-AA *(National Institute on Aging and Alzheimer's Association*) dementia criteria. No further clinical information was available for the subjects. Tables 1a-1D show additional information about the cohorts.

Tables 1A-1D. Information from the Gothenburg (Sweden) cohort and the Barcelona (Spain) cohort. The tables represent the mean ± standard error. *: significantly different (p < 0.05) from the control group with the same APOE genotype.

| **Table 1A** | | | |
|---|---|---|---|
| **Gothenburg (Sweden) cohort** | | | |
| **Control** | | | |
| ***APOE*** | **ε3/3** | **ε3/4** | **All** |
| **N** | 9 | 5 | 14 |
| **Age (years)** | 69±2 | 62±5 | 67±3 |
| **Age (years)** | 60-81 | 44-75 | 44-81 |
| **Women/Men** | 5/4 | 2/3 | 7/7 |
| **Aβ42 in CSF (pg/mL)** | 845±96 | 746±121 | 804±74 |
| **Tau in CSF (pg/mL)** | 317±53 | 303±34 | 312±35 |

| **Table 1B** | | | | |
|---|---|---|---|---|
| **Gothenburg (Sweden) cohort** | | | | |
| **AD** | | | | |
| ***APOE*** | **ε3/3** | **ε3/4** | **ε4/4** | **All** |
| **N** | 15 | 15 | 15 | 45 |
| **Age (years)** | 79±2 | 78±1 | 73±1 | 77±1* |
| **Age (years)** | 62-88 | 69-84 | 63-83 | 62-88 |
| **Women/Men** | 11/4 | 11/4 | 9/6 | 31/14 |
| **Aβ42 in CSF (pg/mL)** | 470±13* | 480±8* | 419±21 | 457±10* |
| **Tau in CSF (pg/mL)** | 816±88* | 917±112* | 731±53 | 840±52* |

| **Table 1C** | | | |
|---|---|---|---|
| **Barcelona (Spain) cohort** | | | |
| **Control** | | | |
| ***APOE*** | **ε3/3** | **ε3/4** | **All** |
| **N** | 5 | 5 | 10 |
| **Age (years)** | 71±2 | 67±52 | 69±2 |
| **Age (years)** | 66-76 | 60-72 | 60-76 |
| **Women/Men** | 1/4 | 2/3 | 3/7 |
| **Aβ42 in CSF (pg/mL)** | 1139±248 | 1010±116 | 1075±131 |
| **Tau in CSF (pg/mL)** | 295±49 | 261±23 | 278±26 |

| **Table 1D** | | | | |
|---|---|---|---|---|
| **Barcelona (Spain) cohort** | | | | |
| **AD** | | | | |
| ***APOE*** | **ε3/3** | **ε3/4** | **ε4/4** | **All** |
| **N** | 10 | 10 | 9 | 29 |
| **Age (years)** | 75±2 | 73±2 | 72±2 | 73±1 * |
| **Age (years)** | 64-84 | 64-83 | 61-85 | 61-85 |
| **Women/Men** | 7/3 | 2/8 | 7/2 | 16/13 |
| **Aβ42 in CSF (pg/mL)** | 607±60* | 543±37* | 493±62 | 549±31* |
| **Tau in CSF (pg/mL)** | 778±94* | 624±62* | 908±81 | 765±50* |

### Western blot

Human CSF samples (10 µL) were denatured at 98°C for 5 minutes and resolved by sodium dodecyl sulphate-polyacrylamide gel electrophoresis (SDS-PAGE) under reducing or non-reducing conditions (determined by the presence or absence of β-mercaptoethanol used in the sample buffer). 12% polyacrylamide gels (Bio-Rad Laboratories, GmbH, Munich, Germany, # 4561046) were used for this study. After electrophoresis, proteins were transferred to nitrocellulose membranes (Bio-Rad Laboratories, GmbH, Munich, Germany). Immunoreactive ApoE bands were detected using either the AB178479 antibody (goat polyclonal; Merck Millipore) or the AB947 antibody (goat polyclonal, Merck Millipore), both common to all ApoE isoforms or, alternatively, by an antibody specific for the ApoE4 isoform (recognizes the Arg112 residue exclusively present in ApoE4 species; mouse monoclonal, Novus Biologicals, NBP1-49529). Subsequentyly the blots were detected using the appropriate conjugated secondary antibodies (IRDye secondary antibodies, LI-COR Biosciences, Lincoln, NE, USA) and images were captured with an Odyssey CLx infra-red imaging system (LI-COR Biosciences). The intensities of the bands were analysed using the LI-COR software (ImageStudio Lite). All samples were analysed in duplicate. Recombinant ApoE3 (Peprotech, ThermoFisher Scientific, #350-12) was included in each blot to serve as a loading reference and to normalize the immunoreactivity signal between blots.

For native polyacrylamide gel electrophoresis (PAGE), the CSF samples were not heated and loaded with NuPage LDS Sample Buffer (ThermoFisher Scientific, NP007) onto nativePage 4-16% gels (ThermoFisher Scientific, BN1002BOX). Buffers were prepared using native PAGE running buffer (ThermoFisher Scientific, BN2001) and native PAGE cathode buffer additive (Thermofisher Scientific, BN2002).

### ApoE immunoprecipitation

The CSF samples (50 µL) were incubated overnight with 100 µL of Dynabeads (Merck Millipore) along with the ApoE antibody 178479 (Merck Millipore). The supernatant was removed, and the beads were washed. They were then resuspended, boiled at 98°C for 5 minutes in SDS-PAGE sample buffer and analysed by Western blotting with the AB947 antibody (Merck Millipore).

### Statistical analysis

All data were analysed using GraphPad Prism (Version 7; GraphPad software, San Diego, CA, USA). The Kolmogorov-Smirnov test was used to analyse the distribution of each variable. ANOVA for parametric variables and the Kruskal-Wallis test for non-parametric variables were used in the comparison between groups. A Student's t-test for parametric variables and a Mann-Whitney U-test for non-parametric variables were used in the comparison between two groups and in determining p-values. Pearson and Spearman tests were used for correlations. The results are given as means ± standard error.

### Example 1. Characterization of ApoE in human CSF

The presence of ApoE species in CSF from controls and AD patients was examined using SDS-PAGE and Western blot under reducing conditions (in the presence of the reducing agent β-mercaptoethanol that breaks disulphide bonds) in samples from a Gothenburg (Sweden) cohort, including subjects with different APOE genotype. In this cohort, AD biomarkers were measured by ELISA, confirming elevated CSF T-tau levels and low Aβ42 levels in AD patient samples (Table 1B).

In all CSF samples, using antibody AB178479, ApoE appears as two distinct immunoreactive bands of ~34 and ~36 kDa (Fig. 1A). Immunoprecipitation with antibody AB178479 and immunoblotting with alternative ApoE antibody AB947 confirmed the identity of both 34 and 36 kDa ApoE monomeric species (Fig. 1B). An ApoE band of ~100 kDa was also observed in CSF samples from AD patients; immunoprecipitation also confirmed the 100 kDa species as ApoE (Fig. 1A, Fig. 1B).

The two distinct bands with different molecular mass could represent different glycoforms of the protein. An enzymatic deglycosylation assay was performed to investigate this aspect. N-deglycosylation did not alter the ApoE band pattern. However, O-deglycosylation simplified the ApoE pattern to a single immunoreactive band, suggesting that O-glycosylation could explain the differences in molecular mass between the 36 and 34 kDa ApoE species (Fig. 1C). The small differences in electrophoretic mobility between glycosylated and deglycosylated ApoE monomers are probably related to the slight carbohydrate mass associated with O-glycosylation, but also to changes in protein shape affecting their migration. Glycosylated proteins are typically more globular because their carbohydrate chains are non-linear, even under reducing conditions. The apparent levels of the 100 kDa ApoE band were not significantly modified after enzymatic deglycosylation, suggesting that these species are also resistant to enzymatic deglycosylation (Fig. 1C).

Even more interestingly, the ~100 kDa ApoE species was observed almost exclusively in CSF samples from AD patients, including samples with APOE ε4/ε4 genotype (Fig. 1A). To further characterize this ApoE species, SDS-PAGE studies were performed under non-reducing conditions to preserve disulphide bonds. Previous studies indicated that ~100 kDa is the molecular mass identified for ApoE homodimers in the brain (Elliott et al., 2010) and CSF (Rebeck et al., 1998), when samples are analysed in the nonreduced state, as opposed to the ~70 kDa predicted. The 100 kDa ApoE band appeared indistinguishable from that observed under reducing conditions in cases of AD, and was also present in control samples, likely representing dimers of disulphide bonds (Fig. 2A). When an ApoE4-specific antibody, NBP1-49529, was used, APOE ε3/ε4 genotype controls did not show any immunoreactivity, whereas immunoreactivity was detected in AD subjects with APOE ε3/ε4 genotype under both reducing and non-reducing conditions (Fig. 2B). This confirms that ApoE4 in samples from AD patients is involved in complexes that form stable 100 kDa species independent of disulphide bonds, representing aberrant/abnormal ApoE aggregates.

The different contribution of the 100 kDa ApoE species in AD cases and controls was first established by estimating the ApoE dimer/monomer balance by native PAGE electrophoresis. Two immunoreactive ApoE bands were observed that likely represent ApoE monomers and dimers, respectively (Fig. 2C). Comparison of the band pattern of amphiphilic protein complex analysis using native PAGE and SDS-PAGE is complicated by the limitations in comparing molecular size determinations with the motilities of hydrophilic molecular weight markers. To address this, we included the resolution of a control sample of CSF denatured by boiling at 95°C for 5 minutes under fully reducing conditions and analysed by native PAGE, along with recombinant ApoE3 protein under native conditions (Fig. 2C). These samples were used to identify the monomeric and dimeric ApoE bands despite the potential difference in estimating the molecular mass of the ApoE complexes resolved under native conditions, SDS-PAGE and Western blot using the AB178479 antibody. Unexpectedly, dimers were detected in CSF from AD cases with APOE ε4/ε4 genotype, whose lack of Cys112 should eliminate their ability to form disulphide bond-dependent complexes. Due to the difficulty in finding control subjects with APOE ε4/ε4 genotype (given the low prevalence of this genotype in the healthy general population), it was not possible to compare AD patients with the APOE ε4/ε4 genotype to control cases with APOE ε4/ε4 genotype. The dimer/monomer ratio of ApoE was compared between CSF samples from control subjects and AD patients, subgrouping the samples by APOE genotype (Fig. 2D). In the control group, the dimer/monomer ratio was significantly lower in the group with the APOE ε3/ε4 genotype (dimer/monomer ratio = 0.38) compared to the group with the ε3/ε3 genotype (dimer/monomer ratio = 2.20; p = 0.006), probably associated with the inability of the ApoE4 isoform to form disulphide-linked dimers. The same situation was found in the AD patient group, where the dimer/monomer ratio decreased as the ε4 allele became more prevalent (ε3/ε3: dimer/monomer ratio = 2.27; ε3/ε4: dimer/monomer ratio = 1.04; ε4/ε4: dimer/monomer ratio = 0.24). For subjects with the APOE ε3/ε3 genotype, the dimer/monomer ratio in the controls was not significantly different from that found in AD subjects. However, for subjects with the APOE ε3/ε4 genotype, the ratio was higher in the AD patient group compared to the controls (p = 0.02). This may be reflecting the accumulation in samples from patients with AD who express ApoE4 of aberrant aggregates in addition to the physiological disulphide bond dimers present only in the controls.

### Example 2. Levels of ApoE species in CSF in AD patients

Given the presence of ApoE aggregates, the levels of the different ApoE species were assessed by SDS-PAGE and Western blot with the AB178479 antibody, a technique that allows for the quantification of individual ApoE species. Therefore, the levels of the 34 kDa, 36 kDa, and 100 kDa ApoE species were evaluated by SDS-PAGE under reducing conditions (Fig. 3A). The 34 kDa ApoE band was significantly increased in AD patients compared to controls (p = 0.003; Fig. 3B). When discriminating by APOE genotype, only the APOE ε3/ε3 genotype was statistically significantly elevated in patients with AD compared to control samples (p = 0.02; Fig. 3C). The same analysis within the APOE ε3/ε4 genotype exhibited lower statistical power due to the small size of the control group; however, a trend towards an increase in 34 kDa ApoE was observed in the AD patients with the APOE ε3/ε4 genotype compared to the controls (p = 0.09; Fig. 3C). The 36 kDa ApoE appeared significantly increased overall in AD patients compared to controls (p = 0.002; Fig. 3D), but not when comparing groups with the same APOE genotype (Fig. 3E).

Although these results indicate a net increase in ApoE in CSF in samples from AD patients, when defining a ratio between the monomeric ApoE species (36 kDa/34 kDa ratio) an imbalance is detected in samples from AD patients, which showed a decrease in the 36 kDa/34 kDa ratio compared to the controls (p = 0.007; Fig. 3F). These differences persisted when separating by APOE genotype (control ε3/ε3: 36 kDa/34 kDa ratio = 1.61 vs AD patients ε3/ε3: 36 kDa/34 kDa ratio = 1.46, p = 0.01; control ε3/ε4: 36 kDa/34 kDa ratio = 1.67 vs AD patients ε3/ε4: ratio = 1.38, p= 0.001; Figure 3G). In the AD patient group, significant differences were also observed between genotypes, with the 36 kDa/34 kDa ratio being significantly lower in APOE ε3/ε3 (p < 0.0001) and ε3/ε4 (p < 0.0001) samples compared to AD patients samples with the ε4/ε4 genotype (36 kDa/34 kDa ratio = 1.64).

As mentioned above, the immunoreactivity of the 100 kDa ApoE species was quite weak in control samples and consequently substantial differences were found between the control samples and those from AD patients (p < 0.0001; Fig. 3H, Fig. 3I). In the AD patient group, the levels of the 100 kDa ApoE species were significantly lower in the APOE ε4/ε4 group compared to the ε3/ε3 (p < 0.0001) and ε3/ε4 (p < 0.0001) groups. Interestingly, among AD patients with the APOE ε4/ε4 genotype, there were 5 samples with low 100 kDa ApoE immunoreactivity that turned out to be younger subjects (AD APOE ε4/ε4 patients with the lowest 100 kDa levels: n = 5, 68 ± 2 years vs cases of AD APOE ε4/ε4 patients with 100 kDa levels similar to other AD cases: n = 10, 76 ± 1 years, p = 0.002). Within this subgroup, ε4/ε4 APOE AD patients, a statistically significant correlation was also observed between the age of the participants and the immunoreactivity of the 100kDa ApoE band (R = 0.622, p = 0.0133).

We evaluated whether the biomarker scores of the CSF samples correlated with the ApoE values obtained. It was found that T-tau correlates with the levels of ApoE species at 34 kDa (p < 0.0001), 36 kDa (p = 0.0009), and 100 kDa (p < 0.0001), as well as with the 36 kDa/34 kDa ratio (p = 0.02) and mean age (p = 0.03). It was observed that the age of the subjects correlated with the 36 kDa/34 kDa ratio (p = 0.02) and the 100 kDa ApoE species (p = 0.0002).

### Example 3. Levels of ApoE species in CSF in AD in a second cohort

The previous data were compared with data from a second independent cohort of CSF samples from Barcelona (see Tables 1C and 1D, Fig. 4A). Similarly to the first cohort, the levels of 34 kDa ApoE were significantly higher in AD patients compared to controls (p = 0.001) and, specifically, this was true only for those with the APOE ε3/ε3 genotype (p = 0.01) (Fig. 4B, Fig. 4C). In contrast to the first cohort, the levels of 36 kDa ApoE were not significantly different between the samples from controls and AD patients, nor between genotypes (Fig. 4D, Fig. 4E). The 36 kDa/34 kDa ratio was again lower in AD patients compared to control samples (p < 0.001, Fig. 4F) and this difference persisted when the samples were separated by genotype (AD patients vs controls for APOE ε3/ε3, p = 0.02 and for APOE ε3/ε4, p = 0.03) (Figure 4G). In this cohort, no significant differences were observed between genotypes in AD patients.

It was observed once again that the levels of 100 kDa ApoE were higher in AD patients compared to controls (p = 0.005; Fig. 5H). When the samples were divided into subgroups by APOE genotype (Fig. 4I), the significant differences between AD patients and controls persisted in the ε3/ε3 group (p = 0.01). No statistically significant result was obtained in the comparison between AD patients and controls for APOE ε3/ε4 (p = 0.050), probably due to the small number of controls. Within the group of patients with AD, APOE ε4/ε4 once again showed significantly lower levels of 100 kDa ApoE compared to ε3/ε3 cases (p = 0.02). The results of this cohort confirm that the 100 kDa ApoE is almost exclusive to AD patients and that AD subjects with APOE ε4/ε4 have a reduced ability to form these 100 kDa complexes.

In this cohort, a significant correlation was detected within the group of AD patients between the age of the subjects and the 100 kDa ApoE species (R = 0.6451, p = 0.0002), indicating a possible association between the age of the subjects and the occurrence of these aggregates of this aberrant ApoE. This association persisted within the AD patient groups with APO ε3/ε3 genotype (R = 0.8133, p = 0.004) and ε3/ε4 genotype (R = 0.7993, p = 0.006).

### Example 4. Analysis of brain extracts from AD patients by Western blot. SNA lectin binding assay

Two distinct collections of frontal cortex and temporal cortex extracts from brains of controls and AD patients were analysed. The extracts were solubilized in a buffer with protease inhibitors (50 mM Tris-HCl pH 7.4, 150 mM NaCl, 5 mM EDTA, 0.5% (w/v) Triton X-100, 1% (w/v) Nonidet P-40, 0.5 mM PMSF and a mixture of protease inhibitors). An SDS-PAGE study was performed with the AB178479 antibody, identifying the 34 and 36 kDa monomers. In late-stage AD patients (Braak V-VI), the 36 kDa monomeric ApoE species almost completely disappeared (Fig. 5). Using an SNA (*Sambucus nigra* agglutinin, with specificity for sialic acid) lectin binding assay coupled with agarose, both monomeric forms of ApoE were found to bind to the SNA lectin in brain samples from control subjects (Fig. 5; the total (Total Ct) and the lectin-bound fraction (Ct Binding to SNA)) are shown, confirming that both species are glycosylated to a greater or lesser extent.

### REFERENCES

Elliott et al. (2010). Apolipoprotein-E forms dimers in human frontal cortex and hippocampus. BMC Neurosci., 11, 23. doi:10.1186/1471-2202-11-23
Rebeck et al. (1998). Structure and functions of human cerebrospinal fluid lipoproteins from individuals of different APOE genotypes. Exp Neurol., 149(1), 175-82. doi:10.1006/exnr.1997.6710

## Claims

1. An *in vitro* method for diagnosing Alzheimer's disease in a subject, comprising:
(a) determining, in a biological sample from said subject, an apolipoprotein E (ApoE)-based biomarker selected from the group consisting of:
(i) presence, amount or concentration of ApoE with a size of 34 kDa in an aggregate of ApoE with a size of 100 kDa;
(ii) dimer/monomer ratio of ApoE detected in a native electrophoresis assay, in subjects with an ApoE3/4 genotype; and
(iii) presence of ApoE dimers detected in a native electrophoresis assay, in subjects with an ApoE4/4 genotype; and
(b) comparing the result of the determination of step (a) with a result obtained in reference subjects, wherein any of the following conditions is indicative of a positive diagnosis of Alzheimer's disease in said subject:
(i) the presence, or an increased amount or concentration of ApoE with a size of 34 kDa in an aggregate of ApoE with a size of 100 kDa, in said biological sample, relative to the reference subjects;
(ii) a higher ratio of ApoE dimers/monomers detected in a native electrophoresis assay, in said biological sample, relative to the reference subjects;
(iii) the presence of ApoE dimers detected by a native electrophoresis assay, in said biological sample.

2. The method according to claim 1, wherein, in step (a) ApoE with a size of 34 kDa is detected using a lectin specific for ApoE with a size of 34 kDa.

3. The method according to claim 1 or 2, wherein, in step (a)(i), the presence of ApoE4 in the 100 kDA ApoE aggregate is determined in a biological sample from a subject with an APOE3/4 or APOE4/4 genotype, with the presence of ApoE4 in the 100 kDa ApoE aggregate being indicative of a positive diagnosis of Alzheimer's disease in said subject.

4. The method according to claim 3, wherein ApoE4 is detected using an ApoE4-specific antibody.

5. The method according to claim 4, wherein the ApoE4-specific antibody is an antibody selected from the group consisting of: antibody NBP1-49529, antibody EPR24181-64 and antibody MABN43.

6. The method according to any one of claims 1 to 5, wherein, in step (a), ApoE is detected with an antibody specific to all ApoE isoforms.

7. The method according to claim 6, wherein said antibody specific for all ApoE isoforms is antibody AB178479 or antibody AB947.

8. The method according to any one of claims 1 to 7, wherein said biological sample is selected from the group consisting of: cerebrospinal fluid, urine, blood, whole blood, plasma, serum, tear, lymphatic fluid, saliva, cells and tissues.

9. The method according to any one of claims 1 to 8, wherein, in the determination of the biomarker in step (a)(i), a technique selected from the group consisting of: gel electrophoresis under reducing conditions, gel electrophoresis under non-reducing conditions, sodium dodecyl sulphate-polyacrylamide gel electrophoresis (SDS-PAGE) under reducing conditions, SDS-PAGE under non-reducing conditions, native electrophoresis, native polyacrylamide gel electrophoresis (PAGE), Western blot, lectin binding assays and enzyme-linked immunoabsorption assay (ELISA) is used.

10. The method according to claim 9, wherein the gel electrophoresis under reducing conditions comprises using the reducing agent β-mercaptoethanol or dithiothreitol.

11. The method according to any one of claims 1 to 10, wherein, in the determination of the biomarker of step (a)(ii) or (a)(iii), said assay is a native polyacrylamide gel electrophoresis (PAGE) assay.

12. A kit for diagnosing Alzheimer's disease, comprising reagents for:
(a) detecting and/or quantifying the amount or concentration of ApoE with a size of 34 kDa in an aggregate of ApoE with a size of 100 kDa, in a biological sample obtained from a subject; or
(b) determining the ratio of ApoE dimers/monomers detected in a native electrophoresis assay, in a biological sample obtained from a subject; or
(c) detecting the presence of ApoE dimers identified in a native electrophoresis assay, in a biological sample obtained from a subject,
wherein the kit comprises at least the AB178479 antibody or the AB947 antibody, specific for all isoforms of ApoE.

13. The kit according to claim 12, further comprising a lectin specific for ApoE with a size of 34 kDa.

14. The kit according to claim 12 or 13, further comprising an ApoE4-specific antibody selected from the group consisting of: the NBP1-49529 antibody, the EPR24181-64 antibody, and the MABN43 antibody.

15. The kit according to any one of claims 12 to 14, further comprising the reducing agent β-mercaptoethanol or dithiothreitol.

16. The kit according to any one of claims 12 to 15, further comprising at least one buffer solution.
